(19) Europäisches Patentamt
European Patent Office
Office européen des brevets

(11) Veröffentlichungsnummer: **0 205 068 B1**

(12) **EUROPÄISCHE PATENTSCHRIFT**

(45) Veröffentlichungstag der Patentschrift: **02.01.92**

(51) Int. Cl.5: **C07D 457/06**, A61K 31/48

(21) Anmeldenummer: **86107482.1**

(22) Anmeldetag: **02.06.86**

Die Akte enthält technische Angaben, die nach dem Eingang der Anmeldung eingereicht wurden und die nicht in dieser Patentschrift enthalten sind.

# (54) Neue Lysergsäureamide, Verfahren zu ihrer Herstellung und diese enthaltende therapeutische Mittel.

(30) Priorität: **10.06.85 DD 277175**

(43) Veröffentlichungstag der Anmeldung:
**17.12.86 Patentblatt 86/51**

(45) Bekanntmachung des Hinweises auf die Patenterteilung:
**02.01.92 Patentblatt 92/01**

(84) Benannte Vertragsstaaten:
**CH DE FR GB IT LI**

(56) Entgegenhaltungen:
**EP-A- 0 029 082**
**EP-A- 0 141 387**
**CH-C- 463 525**
**DE-A- 2 523 743**
**US-A- 3 239 530**

**JOURNAL OF THE CHEMICAL SOCIETY. PERKIN TRANSACTIONS I, 1980, Seiten 902-905; H. ISHII et al.: "Studies on lysergic acid diethylamide and related compounds. Part 8. Structural identification of new metabolites of lysergic acid diethylamide obtained by microbial transformation using Streptomyces Roseochromogenes"**

(73) Patentinhaber: **ARZNEIMITTELWERK DRESDEN GmbH**
**Wilhelm-Pieck-Strasse 35**
**O-8122 Radebeul(DE)**

(72) Erfinder: **Hempel, Roland, Dr.**
**Ernst-Thälmann-Strasse 20**
**DD-8101 Mobschatz(DE)**
Erfinder: **Heidenbluth, Karlheinz, Dr.**
**Dr.-Rudolf-Friedrichs-Strasse 27**
**DD-8122 Radebeul(DE)**
Erfinder: **Dauth, Christoph, Dr.**
**Scheringer Strasse 37**
**DD-8122 Radebeul(DE)**
Erfinder: **Schmidt, Joachim, Prof. Dr. sc. med.**
**Straussstrasse 4**
**DD-8051 Dresden(DE)**
Erfinder: **Bartsch, Reni, Dr.**
**Stephensonstrasse 11**
**DD-8045 Dresden(DE)**
Erfinder: **Rostock, Angelika, Dipl.-Biologe**
**Böttgerstrasse 36**
**DD-8023 Dresden(DE)**
Erfinder: **Hoffmann, Wolfgang, Dr.**
**Ernst-Thälmann-Strasse 33**
**DD-8122 Radebeul(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Erfinder: **Morgenstern, Evelyn, Dipl.-Biologe**
**Richard-Sorge-Strasse 84**
**DD-1034 Berlin(DE)**
Erfinder: **Glusa, Erika, Dr. sc. med.**
**Humboldtstrasse 20**
**DD-5082 Erfurt(DE)**
Erfinder: **Grawert, Werner**
**Freiligrath Strasse 18**
**DD-8122 Radebeul(DE)**

(74) Vertreter: **Puchberger, Rolf, Dipl. Ing. et al**
**Patentanwälte Dipl. Ing. Rolf Puchberger**
**Dipl. Ing. Georg Puchberger Singerstrasse 13**
**A-1010 Wien(AT)**

## Beschreibung

Anwendungsgebiet der Erfindung

Die Erfindung betrifft Lysergsäureamide und Verfahren zur Herstellung neuer Lysergsäureamide, die allgemein zur Therapie verminderter, cerebraler Leistungsfähigkeit, insbesondere der altersbedingten Erscheinungen, Anwendung finden sollen, sowie pharmazeutische Zusammensetzungen.

Charakteristik der bekannten technischen Lösungen

Es ist bekannt, das Lysergsäureamide-Derivate viele unterschiedliche Wirkungen aufweisen, wie Antiserotoninwirkungen (DE-A-2 523 743, US-A-3 239 530, CH-PS-463 525),Behandlung therapieresistenter Migräneformen (EP-A-29 082), rheumatischer Affektionen (CH-PS 463 525), konstriktorische Wirkung auf die glatte Gefäßmuskulatur (CH-PS 463 525), anti-depressive Wirkung und neuroleptische Wirkung (DE-A-2 523 747 und siehe auch Martindale, 28th Edition, 1982, Seite 662, Spalte 1, 4.Absatz). Jedoch sind alle diese Verbindungen nie als gedächtnisfördernde Substanzen (sog.Nootropika) eingesetzt worden.

Auf dem Ergolingebiet werden gegenwärtig zur Entwicklung nootrop wirksamer Substanzen hauptsächlich Lysergolesterstrukturen bearbeitet. Die Einführung des Nicergolins (10$\alpha$-Methoxy-1,6-dimethyl-ergolin-8$\beta$-methanol-(5'-bromnicotinat)) als cerebralen Vasodilatator sowie eigene Ergebnisse mit 1-benzylierten Lysergolestern dokumentieren dies.

Andererseits ist bekannt, daß auch bestimmte Lysergsäureamidstrukturen eine gewisse ZNS-Wirkung entfalten. Abgesehen von dem Halluzinogen Lysergsäurediethylamid (LSD) wurden z.B. auch bei Lysergsäurepyrrolidid und beim Lysergsäuremorpholid psychostimulierende Eigenschaften gefunden.

Weiterhin sind O-acylierte Lysergsäurealkanolamide vom 1-Amino-3-allyloxy-propanol-2 sowie vom Nitroarginінol durch die NL-PS-7 506 276 und BE-PS 829 518 bekannt. Es werden dort neben einem starken und lang anhaltenden Serotoninantagonismus vor allem anti-depressive und neuroleptische Eigenschaften genannt.

Versuche, durch die hier erfindungsgemäß beschriebenen acylierten Lysergsäurealkanolamide zu nootrop wirksamen Verbindungen zu gelangen, wurden bisher noch nicht bekannt.

Ziel der Erfindung

Ziel der Erfindung ist die Bereitstellung neuer nootrop wirksamer Verbindungen, die zur Therapie der verschiedensten Formen von reduzierter cerebraler Leistungsfähigkeit eingesetzt werden können. Damit soll auch gleichzeitig auf der Basis der gewonnenen pharmakologischen Befunde ein Beitrag zur Erweiterung der klassischen Indikationsgebiete der Ergoline geleistet werden.

Darlegung des Wesens der Erfindung

Die Erfindung betrifft neue Lysergsäureamide der allgemeinen Formel

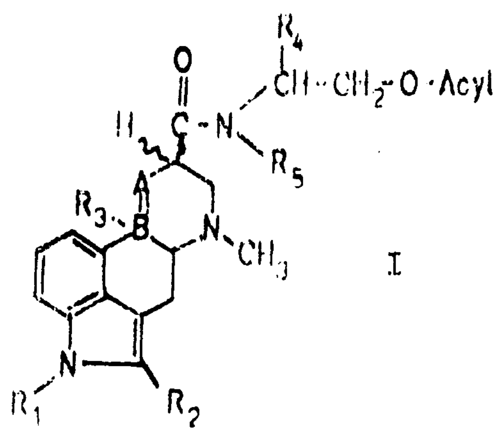

I

worin A...B für die Bindungen steht und die Reste

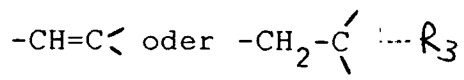

$R_1$ Wasserstoff, niederes Alkyl oder Benzyl, das gegebenenfalls durch Halogen monosubstituiert ist
$R_2$ Wasserstoff oder Halogen
$R_3$ Wasserstoff oder Alkoxy
$R_4$ Wasserstoff oder niederes Alkyl
$R_5$ Wasserstoff oder $-CH_2-CH_2-O-$Acyl jedoch $R_4$ und $R_5$ nicht gleichzeitig Wasserstoff sind

Acyl einen aliphatischen Säurerest bedeuten, und worin je nach Stellung der beiden Substituenten am $C_8$ des Ergolinskeletts, relativ zum Wasserstoffatom am $C_5$, sowohl Lysergsäure- als auch Isolysergsäureform vorliegen kann sowie deren Salze mit physiologisch verträglichen Säuren, und die Beispiele 1f, 1g, 1y, 1k, 1m, 1o, 1p, 1s, 1t und 1u. Die neuen Verbindungen der allgemeinen Formel I werden erfindungsgemäß dadurch erhalten, daß man z.B. die bekannten Alkanolamide der Formeln IV bzw. V gemäß Anspruch 23 mit dem reaktiven Derivat einer Säure (z.B. Säurechlorid oder Säureanhydrid) beispielsweise in Pyridin zur Reaktion bringt. Außerdem kann man zu den neuen Verbindungen der allgemeinen Formel I kommen, wenn man die Verbindungen II nach Überführung in ein reaktives Derivat, vorzugsweise als gemischtes Anhydrid mit Schwefelsäure oder als Säurechlorid-Hydrochlorid gleich mit Aminen der allgemeinen Formel VII gemäß Anspruch 24 zur Reaktion bringt. Als vorteilhaftes Kondensationsverfahren für diese Stufe hat sich außerdem auch das Geiger-König-Verfahren (W.König und R.Geiger, Chem.Ber. 103, (1970) 788) erwiesen. Auf prinzipiell ähnlichem Wege sind natürlich auch die Zwischenprodukte der Formeln IV und V durch Reaktion von Substanzen der Formeln II mit VI, in welcher $R_6$ = Wasserstoffoder $-CH_2-CH_2-OH$ bedeutet, zugänglich, wobei im Falle der Substanzen der Formel V für die weitere Umsetzung zu Formel I vorteilhaft z.B. gleich von Ergometrin bzw. Methylergometrin ausgegangen werden kann.
Die Synthese der O-acylierten Amine gemäß Formel VII kann man vorteilhaft unter Anwendung der in der Peptidchemie üblichen N-Schutzgruppen (vgl. Houben-Weyl, Bd. 15/1) durchführen oder man verfährt nach speziellen Literaturverfahren, wie sie z.B. für das 2,2'-Diacethoxy-diethyl-ammoniumchlorid angegeben sind (C.W.Crane und H.N.Rydon, J.Chem. Soc., 1947, 527).
Die Herstellung der 1-aralkylierten Derivate der Formel V ist in Dimethylformamid/Ätzkali unter Eiskühlung für $R_1$ = H und A ⎓ B gleich

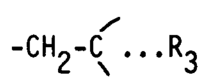

möglich (vgl. EP 29 082).
Eine anschließende Acylierung auf dem üblichen Wege liefert die entsprechende Formel I. Anderenfalls kann auch gleich von den gewünschten 1-substituierten Substanzen gemäß Formel II ausgegangen werden, was besonders im Falle von A ⎓ B gleich

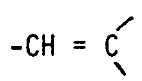

günstig ist.

Zur Einführung der 10α-Alkoxysubstituenten geht man vorteilhaft z.B. von der 10α-Methoxy-9,10-dihydrolysergsäure aus, die auf bekanntem Wege aus dem Methylester leicht zugänglich ist. Man kann jedoch die fotochemische Addition der Alkanole an die 9,10-Doppelbindung auch bei der Formel V durchführen.

Zur Herstellung der 2-halogenierten Derivate gemäß Formel I kann prinzipiell in jeder Verfahrensstufe halogeniert werden. Vorteilhaft läßt sich jedoch die Halogenierung der Esterstruktur gemäß Formel I als letzter Verfahrensschritt, z.B. eine Bromierung in Dichlormethan mittels N-Brom-succinimid, ausführen.

Die erhaltenen erfindungsgemäßen Verbindungen werden vorteilhaft in das Säureadditionssalz einer physiologisch verträglichen Säure überführt. Als übliche physiologisch verträgliche Säuren kommen z.B. Salzsäure, Phosphorsäure oder Maleinsäure, Weinsäure sowie Methansulfonsäure in Betracht. Die Herstellung dieser Säureadditionssalze erfolgt in an sich bekannter Weise durch Mischen der freien Base oder deren Lösungen mit der entsprechenden Säure oder deren Lösungen in einem organischen Lösungsmittel, beispielsweise einem niederen Alkohol, einem niederen Keton oder einem Ether. Zur Verbesserung der

Kristallisation der Salze sind gegebenenfalls auch Mischungen der genannten Lösungsmittel empfehlenswert. Desweiteren kann man pharmazeutisch vertretbare, wäßrige Lösungen der Säureadditionssalze durch Auflösen der freien Base der allgemeinen Formel I in einer wäßrigen Säurelösung gewinnen.

Die Erfindung betrifft auch pharmazeutische Zusammensetzungen, enthaltend ein Lysergsäurederivat der allgemeinen Formel

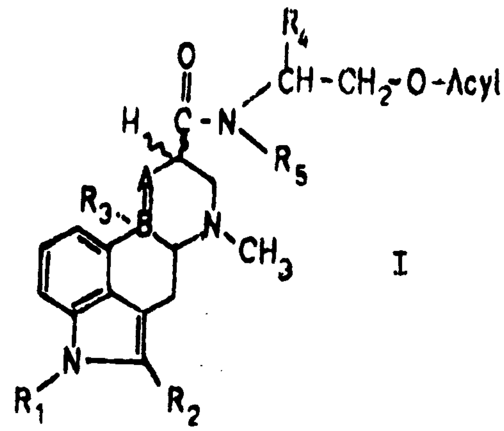

I

oder nach den Beispielen 1f, 1g, 1j, 1k, 1m, 1o, 1p, 1s, 1t und 1u, bzw. eines seiner physiologisch verträglichen Säureadditionssalze als Wirkstoff,insbesondere zur Behandlung von

- postapoplektischen Zuständen
- Minderung kognitiver und mnestischer Leistungen unterschiedlicher Genese
- altersbedingten, hirnorganischen Psychosyndromen
- vaskulär bedingten cerebralen Funktionsstörungen
- hypoxisch, neurotoxisch und traumatisch bedingten cerebralen Funktionsstörungen
- verminderter cerebraler Leistungsfähigkeit bei Alkoholikern
- pathologisch bzw. altersbedingt vermindertem Venentonus.

Die therapeutische Anwendung der neuen Verbindungen erfolgt als Nootropika in Form von Tabletten, Dragees, Tropflösungen, eines Ampullenpräparates oder anderer geeigneter Verabreichungsformen, enthaltend 0,1 bis 10 mg pro Dosierungseinheit eines Lysergsäurederivates der allgemeinen Formel I bzw. eines seiner physiologisch verträglichen Säureadditionssalze als Wirkstoff neben üblichen galenischen Beimengungen, wie z.B. Trägerstoffen, Antioxidantien und Verdünnungsmitteln. Die bevorzugten Zubereitungen sind jedoch Darreichungsformen, die zur oralen Applikation geeignet sind, wie z.B. Dragees oder auch entsprechende Depotformen. Daneben kommen auch parenterale Zubereitungen, wie Injektionslösungen in Betracht.

Die Dosierung der erfindungsgemäßen Substanzen hängt vom Alter, Zustand und Gewicht des Patienten, sowie von der Applikationsform ab. In der Regel wird eine orale Einzeldosis zwischen 1 und 10 mg liegen.

## Pharmakologische Befunde

Die $N_1$-methylierten sowie $N_1$-unsubstituierten Derivate des Lysergsäure- bzw. 9,10-Dihydrolysergsäurediethanolamides sind bereits durch die CH-PS 463 525 bekannt. Man gibt dort einen starken Serotoninantagonismus sowie eine gewisse konstriktorische Wirkung auf die glatte Gefäßmuskulatur an und schlägt diese Substanzen zur Behandlung von vaskulären Kopfschmerzen vor. Aufgrund dieses Wissensstandes ist die von uns im Tierexperiment gefundene, ausgeprägte, nootrope Wirkung der durch Acylierung der Substanz gemäß Formel IV gewonnenen Substanzen gemäß Formel I überraschend. Auch im Falle der Substanz gemäß Formel V kann, obgleich es Hinweise auf cerebrale Aktivität bei einigen 1-benzylierten Ergometrinen gab (EP-A-29 082), eine Wirkungsverstärkung durch Veresterung der Substanz gemäß Formel V nicht als naheliegend betrachtet werden.

Weiterhin konnten bisher keine die therapeutische Anwendung einschränkenden Nebenwirkungen gefunden werden. Interessant ist ferner, daß die bei einigen Ergolinen auftretende α-Adrenolyse hier nur in untergeordnetem Maße vorhanden ist. Außerdem muß hervorgehoben werden, daß es bei bestimmten Verbindungen, wie z.B. 1a, gelang, einige pharmakologisch wertvolle Nebenwirkungen wie beispielsweise

Tonisierung der Venen herauszuarbeiten. Diese zusätzlich zu der nootropen Hauptwirkung auftretenden pharmakologischen Effekte können als willkommene Ergänzung des pharmakologischen Wirkungsprofiles dieser Substanzklasse bei der Therapie cerebraler Schäden vor allem älterer Patienten angesehen werden.

Nachfolgend ist eine Auswahl der an in vivo- und in vitro-Modellen gefundenen pharmakologischen Eigenschaften dieser neuen Verbindungen angegeben.

1. Aktive bedingte Fluchtreaktion

W.Hoffmann und A. Rostock, Pharmazie 38 (1983), 869

Beeinflussung der durch elektrokonvulsive Schocks ausgelösten retrograden Amnesie am Modell einer aktiven bedingten Fluchtreaktion, dargestellt ist die Anzahl der bedingten Reaktionen (%).
$\bar{x} \pm s\bar{x}$, * p<0,05, **p<0,01, Ratte

| Substanz Dosis | 1.Tag | 2.Tag | 3.Tag | 4.Tag |
|---|---|---|---|---|
| Kontrolle i.p. | 33,0±1,5 | 50,0±3 | 64,0±3,1 | 64,0±1,6 |
| | | ** | ** | ** |
| i.p. 1a 3 mg/kg | 33,0±1,5 | 65,0±2,7 | 78,0±1,3 | 84,0±2,2 |
| Kontrolle i.p. | 32,0±6,3 | 48,0±5,7 | 49,0±5,2 | 57,0±5,0 |
| Nicergolin | | | ** | ** |
| i.p. 1mg/kg | 32,0±2,0 | 57,0±5,6 | 77,0±4,2 | 79,0±2,3 |

| | | | | |
|---|---|---|---|---|
| Kontrolle p.o. | 26,0±1,6 | 44,0±3,1 | 57,0±2,6 | 66,0±2,7 |
| | | ** | ** | ** |
| p.o. 11 1 mg/kg | 28,0±2.0 | 60,0±2,6 | 74,0±3,1 | 79,0±3,1 |
| Kontrolle p.o. | 31,0±1,8 | 52,0±2,5 | 58,0±3,6 | 66,0±2,2 |
| | | ** | ** | ** |
| p.o. 1c 3 mg/kg | 30,0±1,5 | 69,0±3,8 | 76,0±3,1 | 82,0±2,0 |
| Kontrolle p.o. | 26,0±1,6 | 44,0±3,1 | 57,0±2,6 | 66,0±2,7 |
| Nicergolin | | ** | ** | * |
| p.o. 1mg/kg | 26,0±1,6 | 61,0±3,8 | 75,0±4,0 | 79,0±5,0 |

2. Schwimmtest nach Porsolt

R.D.Porsolt et al. Arch.Intern. Pharmacodyn. 229,(1977) 327 Beeinflussung der Immobilitätsdauer, Maus ip.

| Substanz | Dosis | Immobilitätsdauer (sec) $\bar{x} \pm s\bar{x}$ |
|---|---|---|
| Kontrolle | - | 226,3 ± 1,4 |
| <u>1 a</u> | 1 mg/kg i.p. | 202,6 ± 1,8 |
| Kontrolle | - | 228,8 ± 1,5 |
| Nicergolin | 1 mg/kg i.p. | 196,7 ± 5,6 |
| Kontrolle | - | 226,1 ± 1,2 |
| <u>1 1</u> | 1 mg/kg i.p. | 199,4 ± 2,3 |
| Kontrolle | - | 228,2 ± 1,1 |
| <u>1 c</u> | 3 mg/kg p.o. | 203,6 ± 5,9 |
| <u>1 e</u> | 3 mg/kg p.o. | 210,0 ± 3,8 |

<u>3. Beeinflussung transcallosal ausgelöster Potentiale</u>

Methodik in Anlehnung an C.Giurgea und F.Moyersoons, Arch.Int.Pharmacodyn.199,(1972) 67

Die Untersuchung wurde an wachen, im stereotaktischen Apparat fixierten weiblichen Wistarratten durchgeführt.

Applikation: i.p. VW = Vorwert * $p < 0{,}05$

| Substanz | Dosis mg/kg | | Amplitudenbeeinflussung b.mittl.Reizintensität µV($\bar{x} \pm s\bar{x}$) neg.Welle | b.mittl.Reizintensität pos.Welle | b.max.Reizintensität µV($\bar{x} \pm s\bar{x}$) neg.Welle | b.max.Reizintensität pos.Welle |
|---|---|---|---|---|---|---|
| 1 a | 1 | VW | 0,18±0,02 | 0,19±0,02 | 0,34±0,03 | 0,38±0,03 |
| | | 60' | 0,23±0,03 | *0,26±0,03 | *0,40±0,03 | 0,42±0,03 |
| | | 120' | *0,25±0,03 | *0,27±0,03 | 0,40±0,02 | 0,41±0,02 |
| | | 180' | 0,23±0,03 | *0,25±0,02 | 0,39±0,02 | 0,42±0,02 |
| Nicergolin | 1 | VW | 0,23±0,02 | 0,23±0,02 | 0,39±0,02 | 0,39±0,02 |
| | | 60' | 0,24±0,02 | 0,24±0,01 | 0,41±0,03 | 0,40±0,02 |
| | | 120' | 0,23±0,02 | 0,22±0,02 | 0,38±0,02 | 0,36±0,02 |
| | | 180' | 0,21±0,01 | 0,19±0,02 | 0,39±0,04 | 0,39±0,04 |

4. SFT-Test (Spinal cord fixation time)

C.Giurgea und F.Mouravieff-Lesmisse Arch.Intern.Pharmacodyn.191 (1971) 279

Es ist die Anzahl der Tiere mit persistierender Asymmetrie zur Gesamtzahl der eingesetzten Ratten aufgeführt.

| Substanz | Dosis ip. mg/kg | Ergebnis |
|---|---|---|
| 1 a | 2 | 5/10 |
| Nicergolin | 1 | 2/10 |
| | 3 | 4/6 |

5. Noradrenalin-Antagonismus

Noradrenalin-Antagonismus am vas deferens des Meerschweinchens

| Substanz | Konzentration mol/l | Hemmung ($\bar{x} \pm s\bar{x}$) in % |
|---|---|---|
| 1 a | $10^{-7}$ | 4,4 ± 9,7 |
| | $10^{-6}$ | 14,7 ± 17 |
| | $10^{-5}$ | 60,7 ± 66,7 |
| Nivergolin | $ED_{50} = 4,3 \times 10^{-8}$ mol/l | |

6. Untersuchungen an isolierten menschlichen Gefäßpräparaten

(Vena und Arteria femoralis)

Glusa, E. und F. Markwardt, Pharmacolgy 24 (1982) 287 sowie Naunyn-Schmiedebergs-Arch. Pharmacol. 323 (1983) 101

| Substanz | Gefäßpräparat | Konzentration µ mol/l | %-Kontraktion bezogen auf NA | Anzahl der Gefäßpräparate |
|---|---|---|---|---|
| 1 h | Vene | 3,9 | 106 ± 33 | 3 |
| | | 1,0 | 15 | 1 |
| | Arterie | 3,9 | 38 ± 7,5 | 3 |
| 1 a | Vene | 3 | 70 ± 20 | 4 |
| | | 1 | 65 ± 15 | 3 |
| | | 0,1 | 45 ± 17 | 5 |
| | Arterie | 3 | 11 ± 10 | 5 |
| Dihydroergotamin | Vene | 1 | 61 ± 24 | 6 |
| | | 0,1 | 52 ± 18 | 6 |
| | Arterie | 1 | 8,8 ± 8 | 4 |

Ausführungsbeispiele

Beispiel 1

9,10-Dihydrolysergsäure-bis-(β-acetoxyethyl)amid 1a

Eine Mischung von 5 g 9,10-Dihydrolysergsäurediethanolamid und 6,6 ml Acetanhydrid in 150 ml Pyridin wird 5 h bei Raumtemperatur gerührt. Zur Aufarbeitung gibt man 50 ml Wasser hinzu, rührt noch 15 min. und bringt im Vakuum zur Trockene. Der viskose Rückstand wird an der 10-fachen Menge Kieselgel mit Chloroform + 1% Methanol chromatographiert. Nach ca. 500 ml Eluat wird der Methanolgehalt auf 3% erhöht. Man erhält 5,7 g (92% der Theorie) einer glasig erstarrten Masse. Nach Umkristallisation aus Aceton werden

Fp: 142,3 - 143,0°C (Stäbchen)

$(\alpha)_D^{20}$ = -50.0° (c = 0,5% in Chloroform) bestimmt.

MS (m/e): 441 ($M^+$), 381, 389, 321, 296, 252, 225, 209, 184, 167,154.

Monotartrat: $C_{24}H_{31}N_3O_5$. 1/2 $C_4H_6O_6$ (MG: 516,2)

Fp. 171 - 173°C (Methylethylketon/Methanol)

$(\alpha)_D^{20}$ = 70,5° (c = 0,2% in Pyridin)

Auf analoger Weise wurden erhalten:

- 9,10-Dihydrolysergsäure-bis-(ß-bezoylethyl)-amid 1g
  Citrat: Fp. 102,5 - 104,6°C,
  $(\alpha)_D^{20}$ = -59,1° (c = 1% in Methanol)
- 1-(3'-Fluorbenzyl)-9,10-dihydrolysergsäure-bis(ß-3''-trifluormethylbenzoylethyl)amid 1k
  Mesylat: Fp. 147,9 - 150,4°C
- 1-(3'-Fluorbenzyl)-9,10-dihydrolysergsäure-bis-(ß-acetoxyethyl)-amid 1l
  Bitartrat: Fp. 82,8 - 84,9°C
  $(\alpha)_D^{20}$ = -48,0° (c = 1% in Methanol)
- 1-Methyl-9,10-dihydrolysergsäure-bis-(ß-acetoxyethyl)-amid 1c
  Bitartrat: Fp. 91 - 92°C
  Base: $(\alpha)_D^{20}$ = -22,3° (c = 0,5% in Methanol)

- 1-Methyl-9,10-dihydrolysergsäure-bis-(ß-propionylethyl)-amid 1e
  Bitartrat: Fp. 77,8 - 81,8°C
  $(\alpha)_D^{20}$=-46,2° (c = 1% in Methanol)

Beispiel 2

2-Brom-9,10-dihydrolysergsäure-bis($\beta$-acetoxyethyl)-amid 1h

Zu einer Lösung von 11 g 9,10-Dihydrolysergsäure-bis-($\beta$-acetoxyethyl)amid in 600 ml Dichlormethan tropft man unter Rühren und Rückflußkochen innert 5 min eine Suspension von 6,05 g N-Brom-Succinimid in 150 ml Dichlormethan. Danach kocht noch 75 min am Rückfluß, kühlt ab und schüttelt zweimal mit 500 ml 10%iger Sodalösung aus. Anschließend wird die organische Phase zweimal mit 500 ml Wasser gewaschen, über Natriumsulfat getrocknet und im Vakuum zur Trockene gebracht. Man erhält 7,5 g hellbraunen Schaum, der in 10 ml Dichlormethan aufgenommen wird und nach Stehen über Nacht bei + 3°C 0,5 g reines Endprodukt als hellgraues Pulver von Fp.: 227 - 228°C liefert. Das Filtrat wird konzentriert und an der 20-fachen Menge Kieselgel mittels Dichlormethan chromatographiert. Nach 200 ml Eluat werden 2% Methanol zugesetzt. Man erhält noch 5,8 g Base, entsprechend 49% der Theorie.

MS (m/e): 521/519 ($M^+$), 461, 447, 388, 332, 317, 303, 289, 261, 147, 232, 224, 215, 190, 171, 154.
Monotartrat: $C_{24}H_{30}N_3O_5Br$ . 1/2 $C_4H_6O_6$ (MG : 595)
Fp.: 168,5 - 171°C (Aceton)
$(\alpha)_D^{20}$ = -75,4° (c = 0,1% in Wasser)

Beispiel 3

9,10-Dihydrolysergsäure-bis-($\beta$-acetoxyethyl)-amid 1a

Eine Mischung von 3,15 g Diethanolamin und 3,24 g Trimethylchlorsilan in 45 ml wasserfreiem Chloroform wird auf dem Ölbad zum Sieden erhitzt. Nach etwa 5 min läßt man etwas abkühlen und gibt 3,03 g Triethylamin, gelöst in 5 ml Chloroform, dazu. Daraufhin kocht man noch 30 min am Rückfluß und kühlt dann auf Raumtemperatur ab. Man läßt anschließend 5,65 ml Acetanhydrid zufließen und rührt noch 3 h nach. Nach Stehen über Nacht alkalisiert man vorsichtig mit 5%iger Sodalösung, wäscht mit wenig

Wasser und trocknet über Natriumsulfat. Die nach dem Konzentrieren der organischen Phase erhaltene ölige Substanz wird anschließend nach dem Geiger-König-Verfähren mit 9,10-Dihydrolysergsäure umgesetzt. Dazu werden 2,8 g 9,10-Dihydrolysergsäure in einer Mischung aus 25 ml Dimethylformamid und 25 ml Methylenchlorid suspendiert und nach Abkühlung auf -10°C zunächst mit 4,06 g Hydroxybenzotriazol versetzt. Nach 15 min Rühren werden 2,24 g Dicyclohexylcarbodiimid zugegeben und nachfolgend bei 0°C der oben erhaltene ölige Rückstand gelöst in 5 ml des genannten Lösungsmittelgemisches. Nach 24 h Rühren bei Raumtemperatur wird abgesaugt und das erhaltene Filtrat im Vakuum zur Trockene gebracht. Der Rückstand wird mit 15 ml 5%iger Weinsäure versetzt.Vom Ungelösten wird abgesaugt und das erhaltene Filtrat zweimal mit 10 ml Methylenchlorid extrahiert. Darauf alkalisiert man mit gesättigter Sodalösung und extrahiert dreimal mit 15 ml Essigester. Nach der üblichen Aufarbeitung des Essigesterextraktes wird ein Produkt gewonnen, das mit dem nach Beispiel 1 erhaltenen identisch ist.

Beispiel 4

10α-Methoxy-9,10-dihydrolysergsäure

Zu einer Lösung von 25,2 g Kaliumhydroxid in einem Gemisch von 345 ml 96%igem Ethanol und 175 ml Wasser werden 13 g 10α-Methoxy-9,10-dihydrolysergsäuremethylester gegeben. Anschließend wird diese Mischung 1,5 h unter Rückflußkochen gerührt. Darauf saugt man ab und bringt das Filtrat zur Trockene. Der erhaltene Rückstand wird in 255 ml Wasser aufgenommen und mit 20%iger Schwefelsäure auf pH 6,9 eingestellt. Nach Stehen über Nacht bei +4°C werden 11,7 g (94,4% der Theorie) 10α-Methoxy-9,10-dihydrolysergsäure als weißes Pulver erhalten.

$C_{17}H_{20}O_3N_2$ (MG: 300,1)

Fp.: 260°C (unter Zersetzung)

$$(\alpha)^{20}_{546} = -14,9^{\circ} \text{ (c = 0,5\% in Methanol)}$$

Gehalt: 86,0%

MS (m/e): 300 ($M^+$), 268, 250, 224, 221, 207, 192, 181, 167, 154.

Beispiel 5

1-Benzyl-10α-methoxy-9,10-dihydrolysergsäure

In einer speziellen Apparatur mit Trockeneiskühltasche und Ableitungsrohr werden nach Füllen der Kühltasche mit Aceton/Trockeneis 200 ml Ammoniak kondensiert. Danach gibt man 180 mg Eisen-(III)-nitrat und anschließend portionsweise 1,2 g Natrium in dem Maße zu, wie die auftretende Blaufärbung wieder verschwindet. Anschließend trägt man 5,22 g 10α-Methoxy-9,10-dihydrolysergsäure ein, rührt 10 min und läßt dann eine Lösung von 12 g Benzylbromid in 10 ml Ether rasch zufließen. Man rührt noch 10 min am Rückfluß und arbeitet dann wie folgt auf:

Nach völligem Abdestillieren des Ammoniaks wird der gewonnene Trockenrückstand zwischen 250 ml Ether und 400 ml Wasser verteilt. Nach Filtration und Phasentrennung wird die wäßrige Phase mit 250 mg Aktivkohle versetzt und 10 min bei Raumtemperatur gerührt. Anschließend wird abgesaugt und das Filtrat mit 50%iger Essigsäure unter Verwendung eines pH-Meßgerätes auf einen pH-Wert von 6,3 eingestellt. Nach Umkristallisation aus Isopropanol erhält man die gewünschte Säure als hellgraues Pulver mit einem Schmelzpunkt von 169,9 -173,3°C.

$C_{24}H_{26}O_3N_2$ (MG: 390,1)

Gehalt: 90,5%

$$(\alpha)^{20}_{546} = -23,0^{\circ} \text{(c = 0,5\% in Methanol)}$$

Beispiel 6

10α-Methoxy-9,10-dihydrolysergsäurediethanolamid

7,76 g 10α-Methoxy-9,10-dihydrolysergsäure und 1,46 g gemörsertes Kaliumhydroxid werden in 100 ml

Methanol gelöst und unter Vakuum zur Trockene gebracht. Nachdem dieser Vorgang wiederholt wurde, löst man den Rückstand in 45 ml Methanol, gibt 520 ml getrocknetes Dimethylformamid hinzu und konzentriert diese Mischung bei 45°C im Vakuum auf etwa 80 ml. Danach fügt man unter Eiskühlung 45 ml Dimethylformamid-Schwefeltrioxid-Komplexlösung (F = 1,22) hinzu und rührt 20 min. Anschließend wird diese Mischung in eine Lösung von 13,6 g Diethanolamin in 28 ml Wasser eingegossen und abermals 20 min gerührt. Zur Aufarbeitung werden noch 12 ml Wasser zugesetzt, mit konzentrierter Ammoniak-Lösung alkalisiert und in Portionen mit insgesamt 600 ml Essigester ausgerührt. Nach Waschen, Trocknen und Konzentrieren des Essigesterextraktes werden 12 g des gewünschten Produktes als hellbrauner Schaum erhalten, der ohne weitere Reinigung zur Acetylierung verwendet werden kann. (MS (m/e): 387 ($M^+$), 369, 356, 355, 268, 223, 201, 184, 167, 154.

Beispiel 7

10α-Methoxy-9,10-dihydrolysergsäure-bis-(β-acetoxyethyl)-amid 1d

Eine Suspension von 3 g 10α-Methoxy-9,10-dihydrolysergsäurediethanolamid in einer Mischung von 18 ml getrocknetem Pyridin und 42 ml getrocknetem Chloroform wird nach Zugabe von 9,45 ml Acetanhydrid 5h bei Raumtemperatur gerührt, wonach eine klare Lösung vorliegt. Diese gießt man in 20 ml Wasser, alkalisiert mit Sodalösung und trennt die Phasen. Anschließend wird noch zweimal mit je 30 ml Chloroform extrahiert. Die vereinigten organischen Extrakte werden gewaschen, über Natriumsulfat getrocknet und im Vakuum zur Trockene gebracht. Der erhaltene schaumige Rückstand wird an der 10-fachen Menge Kieselgel mit Chloroform +3% Methanol chromatographiert.

MS (m/e): 471 ($M^+$), 440, 285, 250, 223, 207, 184, 167, 154. Bitartrat: $C_{25}H_{33}N_3O_6$ . $C_4H_6O_6$ (MG: 621,1)

Fp. 97,8 - 98,3°C (Aceton)

$$(\alpha)^{20}_{578} = -7{,}7^{o} (c=1\% \text{ in Methanol})$$

Auf analoge Weise wurden erhalten:

- 1-Benzyl-10α-methoxy-9,10-dihydrolysergsäure-bis-(ß-3'-chlorbenzoylethyl)-amid 1j
  Hydrochlorid: Fp. 93,7 - 97,2°C $(\alpha)^{20}_{D} = -8{,}7^{o}$ (c = 1% in Methanol)

- 10α-Methoxy-9,10-dihydrolysergsäure-bis-(ß-5'-bromnicotinyl-ethyl)-amid 1f
  Mesylat: Fp. 116,5 - 119°C

Beispiel 8

10α-Methoxy-9,10-dihydroergometrin

In einer speziellen Bestrahlungsapparatur (Quarzstrahler S 180 R, VEB EGS Zella-Mehlis) werden 7,5 g Ergometrin, gelöst in einer Mischung aus 380 ml Methanol und 40 ml konz. Schwefelsäure, 2,5 h bei Raumtemperatur unter Stickstoffspülung bestrahlt. Danach wird die Reaktionslösung in 450 ml Eiswasser eingerührt, vorsichtig mit konz. Ammoniak-Lösung alkalisiert und dreimal mit 400 ml Essigester extrahiert. Die vereinigten organischen Phasen werden über Natriumsulfat getrocknet und im Vakuum eingeengt. Mittels Methanol/Ether erhält man eine rosa gefärbte amorphe Substanz.

10 g dieser Substanz werden 30 min bei 40°C in 40ml Aceton, das 10% Methanol enthält, gerührt. Das mit

Aceton und Ether gewaschene Produkt ist dann als Rohsubstanz für weitere Derivatisierungen geeignet. Zur Feinreinigung von 10α-Methoxy-9,10-dihydroergeometrin wird mit Aceton + 12% Methanol an der 15-fach Menge Kieselgel chromatographiert.

Bitartrat: $C_{20}H_{27}O_3N_3 . C_4H_6O_6$ (MG: 507,4)
Fp.: 211 - 211,5°C
(Stäbchen aus Methanol/Aceton) $(\alpha)_D^{20} = \pm 0°$(c = 0,5% in Methanol)
Bei langsamer Aufheizung erfolgt ab 175°C Zersetzung.

Beispiel 9

1-Methyl-10α-methoxy-9,10-dihydroergometrin

Eine Suspension von 3,88 g fein gemörsertem Natriumhydroxid in 40 ml Dimethylformamid wird 10 min gerührt und anschließend mit 5 g 10α-Methoxy-9,10-dihydroergometrin versetzt. Nach weiteren 20 min Rühren fügt man unter Kühlung mit Naßeis 2,43 g Methyljodid, gelöst in 5 ml Dimethylformamid,hinzu und rührt noch 45 min unter Eiskühlung. Darauf gießt man in 25 ml Wasser und konzentriert diese Mischung im Vakuum. Der Rückstand wird in 100 ml 20%iger Weinsäure gelöst. Nach Absaugen des Niederschlages wird das erhaltene Filtrat zweimal mit 50 ml Methylenchlorid extrahiert. Nun bringt man die wäßrige Phase mit konzentrierter Ammoniak-Lösung auf pH 10-11 und schüttelt dreimal mit je 100 ml Essigester aus. Die vereinigten Essigesterestrakte werden über Natriumsulfat getrocknet und am Vakuumrotationsverdampfer bei 40°C zur Trockene gebracht. Anschließend chromatographiert man diesen Rückstand an der 15-fachen Menge Kieselgel mit Chloroform + 3,5% Methanol. Nach Umkristallisation aus Chloroform und Aceton jeweils unter Methanolzusatz erhält man nach Überführung in das weinsaure Salz 1-Methyl-10α-methoxy-9,10-dihydroergometrin-bitartrat.

$C_{21}H_{29}O_3N_3 . C_4H_6O_6$ (MG: 521,4)
Fp.: 154 - 156°C $(\alpha)_D^{20}$ = 3,9° (c = 1% in Methanol)

Beispiel 10

1-Methyl-10α-methoxy-9,10-dihydrolysergsäure-(+)-propanolamid-(2)-acetat 1r

Zu einer Lösung von 3,21 g 1-Methyl-10α-methoxy-9,10-dihydroergometrin in 45 ml Pyridin tropft man unter Rühren 9,6 ml Acetanhydrid und läßt anschließend noch 3 h bei Raumtemperatur rühren. Nach Stehen über Nacht werden 10 ml Wasser zugesetzt und danach die Mischung im Vakuum konzentrier. Durch nachfolgende Säulenchromatographie an der 10-fachen Menge Kieselgel mit Chloroform +3% Methanol wird ein hellgelber Schaum erhalten, der nach Überführung in das Bimaleinat und Umkristallisation aus Aceton/Ether das Endprodukt als weißes Pulver liefert.

Base: MS (m/e): 413 ($M^+$), 382, 322, 297, 281, 265, 237, 221, 213, 199, 181, 168
Bimaleinat: $C_{23}H_{31}O_4N_3 . C_4H_4O_4$ (MG: 529,5)
Fp.:143,5 - 146,5°C

$$(\alpha)_{578}^{20} = -17{,}9^{\circ} \text{ (c=1\% in Methanol)}$$

Auf analoge Weise wurden erhalten:

- 9,10-Dihydrolysergsäure-(+)-propanolamid-(2)-3'-fluorbenzoat 1o
Bitartrat: Fp. 133,2 - 135,6°C $(\alpha)_D^{20}$ = - 18,8° (c = 0,5% in Methanol)

- 9,10-Dihydrolysergsäure-(+)-propanolamid-(2)-4'-chlorphenoxy-acetat 1p

Monotartrat: Fp. 114,3 - 117,1°C $(\alpha)_D^{20}$= - 30,1° (c=0,25% in Wasser)

- 10α-Methoxy-9,10-dihydrolysergsäure-(+)-propanolamid-(2)-propionat 1q
  Bitartrat: Fp. 111,3 - 113,7°C $(\alpha)_D^{20}$= -4,6° (c = 1% in Methanol)

- 10α-Methoxy-9,10-dihydrolysersäure-(+)-propanolamid-(2)-acetylsalicylat 1s
  Bitartrat: Fp. 116,5 - 120,1°C $(\alpha)_D^{20}$= +8,9° (c = 1% in Methanol)

- 10α-Methoxy-9,10-dihydrolysergsäure-(+)-propanolamid-(2)-3'-chlorbenzoat 1t
  Bitartrat: Fp. 130,1 - 133,3°C $(\alpha)_D^{20}$ = +17,0° (c = 1% in Methanol)

- 10α-Methoxy-9,10-dihydrolysergsäure-(+)-butanolamid-(2)-2'-fluorbenzoat 1u
  Bitartrat: Fp. 123,5 - 125,3°C

Beispiel 11

d-Lysergsäure-bis-(β-acetoxyethyl)-amid 1h/1i

Die Herstellung des Lysergsäurediethanolamids wie in der CH-PS 463 525 beschrieben oder auch nach den sonst in der Ergolinchemie üblichen Amidierungsverfahren,wie beispielsweise der Dimethylformamid-schwefeltrioxid-Methode, erfolgen. Der nach Aufarbeitung der Acetylierung, durchgeführt analog Beispiel 1, anfallende braune Schaum wird an der 15-fachen Menge Kieselgel mit Chloroform + 1% Methanol chromatographiert. Nach ca. 300 ml Eluat erhöht man den Methanolgehalt auf 2% und wäscht darauf eine Zone ins Filtrat, die nach dem Einengen mit acetonischer Weinsäure als Tartrat ausgefällt werden kann. Weitere Elution liefert eine zweite Zone, die ebenfalls als Tartrat isoliert wurde, jedoch nur sehr schwer zur Kristallisation zu bringen war.

1. Zone: d-Lysergsäure-bis-(β-acetoxyethyl)-amid- (MG: 514,1) monotartrat
$C_{24}H_{29}N_3O_5$ . 1/2 $C_4H_6O_6$
Fp. 126 - 128,5°C (Aceton) $(\alpha)_D^{20}$ = +14,9° (c = 0,5% in Methanol)

2. Zone: d-Isolysergsäure-bis-(β-acetoxyethyl)- (MG: 589,1) amid-bitartrat
$C_{24}H_{29}N_3O_5$ . $C_4H_6O_6$
Fp. 83,5 - 86,5°C (Aceton/Ether) $(\alpha)_D^{20}$ = +160,5° (c = 0,12% in Methanol)

Auf analoge Weise wurden erhalten:

- Lysergsäure-bis-(ß-3'-fluorbenzoylethyl)amid 1m
  Mesylat: Fp. 71,5 - 74,1°C $(\alpha)_D^{20}$= -31,2° (c = 0,5% in Methanol)

- 1-Methyl-lysergsäure-bis-(ß-acetoxyethyl)-amid 1n
  Bitartrat: Fp. 77,8 - 80,4°C $(\alpha)_{546}^{20}$= +16° (c =0,25% in Methanol)

Formelblatt

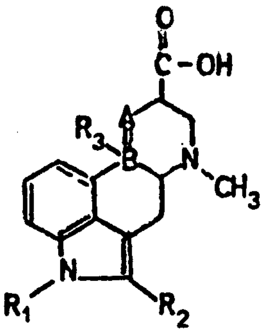

II

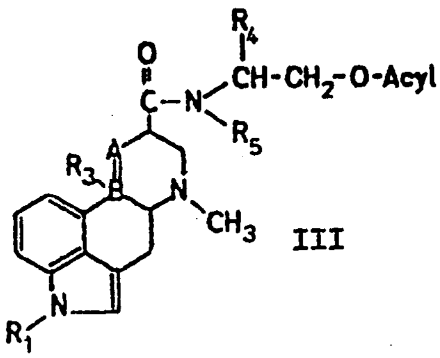

III

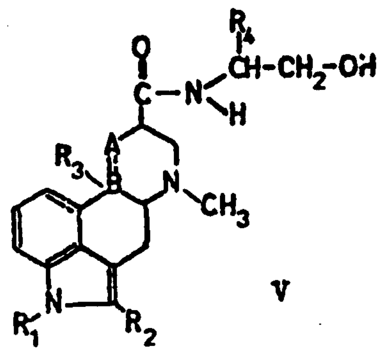

V

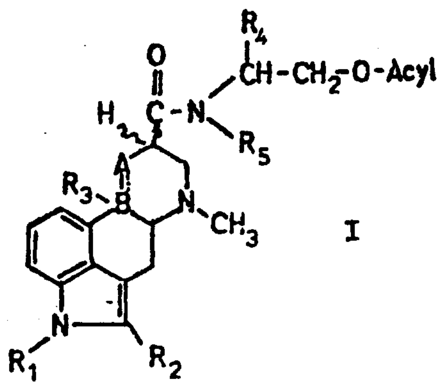

I

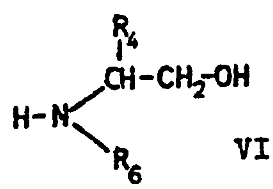

VI

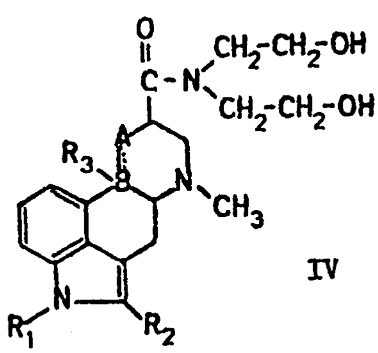

IV

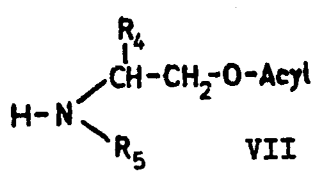

VII

## Patentansprüche

**1.** Lysergsäureamide der allgemeinen Formel

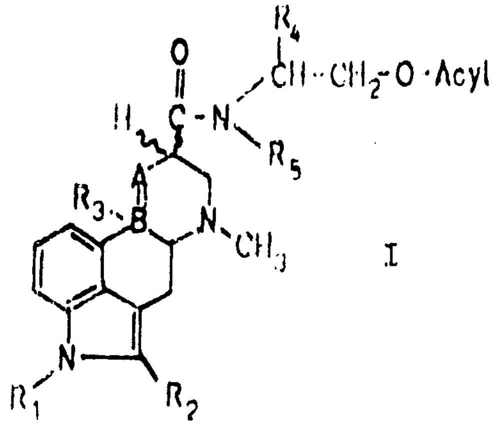

I

worin A...B für die Bindungen

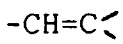 oder 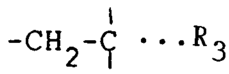

steht und die Reste

- $R_1$ Wasserstoff, niederes Alkyl oder Benzyl, das gegebenenfalls durch Halogen monosubstituiert ist
- $R_2$ Wasserstoff oder Halogen
- $R_3$ Wasserstoff oder Alkoxy
- $R_4$ Wasserstoff oder niederes Alkyl
- $R_5$ Wasserstoff oder $-CH_2-CH_2-O-Acyl$ jedoch $R_4$ und $R_5$ nicht gleichzeitig Wasserstoff sind

Acyl einen aliphatischen Säurerest bedeuten, und worin je nach Stellung der beiden Substituenten am $C_8$ des Ergolinskeletts, relativ zum Wasserstoffatom am $C_5$, sowohl Lysergsäure- als auch Isolysergsäureform vorliegen kann sowie deren Salze mit physiologisch verträglichen Säuren.

**2.** 9,10-Dihydrolysergsäure-bis-(β-acetoxyethyl)-amid gemäß Anspruch 1.

**3.** 2-Brom-9,10-dihydrolysergsäure-bis-(β-acetoxyethyl)-amid gemäß Anspruch 1.

**4.** 1-Methyl-9,10-dihydrolysergsäure-bis-(β-acetoxyethyl)-amidgemäß Anspruch 1.

**5.** 10α-Methoxy-9,10-dihydrolysergsäure-bis-(β-acetoxyethyl)-amid gemäß Anspruch 1

**6.** 1-Methyl-9,10-dihydrolysergsäure-bis-(β-propionylethyl)-amid gemäß Anspruch 1.

**7.** Lysergsäure-bis-(β-acetoxyethyl)-amid gemäß Anspruch 1.

**8.** Isolysergsäure-bis-(β-acetoxyethyl)-amid gemäß Anspruch 1.

**9.** 1-(3'-Fluorbenzyl)-9,10-dihydrolysergsäure-bis-(β-acetoxyethyl)-amid gemäß Anspruch 1.

**10.** 10α-Methoxy-9,10-dihydrolysergsäure-(+)-propanolamid-(2)-propionat gemäß Anspruch 1.

**11.** 1-Methyl-10α-methoxy-9,10-dihydrolysergsäure-(+)-propanolamid-(2)-acetat gemäß Anspruch 1.

**12.** 1-Methyl-lysergsäure-bis-(β-acetoxyethyl)-amid gemäß Anspruch 1.

**13.** 9,10-Dihydrolysergsäure-(+)-propanolamid-(2)-4'-chlorphenoxyacetat.

**14.** 9,10-Dihydrolysergsäure-bis(β-benzoylethyl)-amid.

**15.** 9,10-Dihydrolysergsäure-( + )-propanolamid-(2)3'-fluorbenzoat.

**16.** Lysergsäure-bis-(β-3'-fluorbenzoylethyl)-amid.

**17.** 1-Benzyl-10α-methoxy-9,10-dihydrolysergsäure-bis-(β-3'-chlorbenzoylethyl)-amid.

**18.** 1-(3'-Fluorbenzyl)-9,10-dihydrolysergsäure-bis-(β-3''-trifluormethylbenzoylethyl)-amid.

**19.** 10α-Methoxy-9,10-dihydrolysergsäure-( + )-propanolamid-(2)-acetylsalicylat.

**20.** 10α-Methoxy-9,10-dihydrolysergsäure-( + )-propanolamid-(2)-3'-chlorbenzoat.

**21.** 10α-Methoxy-9,10-dihydrolysergsäure-( + )-butanolamid-(2)-2'-fluorbenzoat.

**22.** 10α-Methoxy-9,10-dihydrolysergsäure-bis-(β-5'-bromnicotinylethyl)-amid.

**23.** Verfahren zur Herstellung neuer Lysergsäureamide der allgemeinen Formel I nach Anspruch 1, oder einem der Ansprüche 13-22,dadurch gekennzeichnet, daß man eine entsprechend substituierte Lysergsäure der allgemeinen Formel

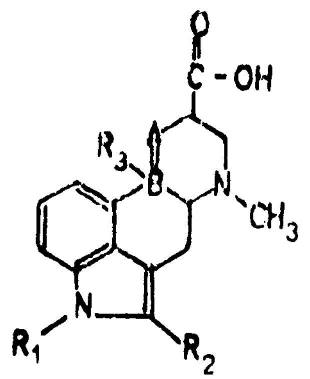

II

mittels der üblichen Methoden der Ergolinchemie über eine aktivierte Form mit einem Amin der allgemeinen Formel

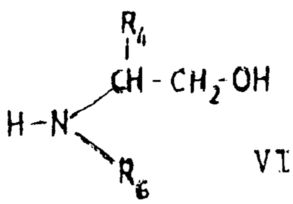

VI

zunächst zu den Zwischenprodukten der allgemeinen Formeln

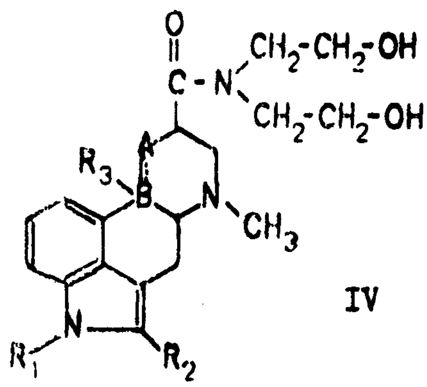

IV

bzw.

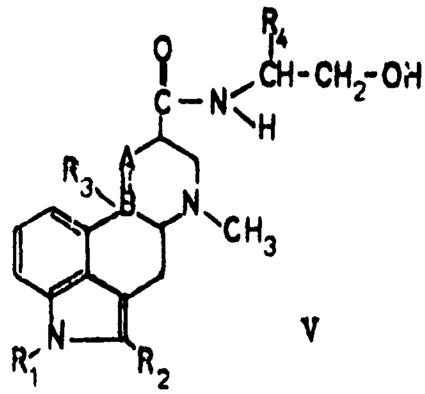

V

umsetzt und diese anschließend nach bekannten Verfahren in eine Verbindung der allgemeinen Formel I überführt.

**24.** Verfahren zur Herstellung neuer Lysergsäureamide der allgemeinen Formel I nach Anspruch 1, oder einem der Ansprüche 13-22,dadurch gekennzeichnet, daß man aus einer entsprechend substituierten Lysergsäure der allgemeinen Formel II über eine aktivierte Form mit einem Amin der allgemeinen Formel

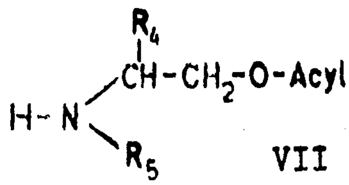

VII

eine neue Verbindung der allgemeinen Formel I gewinnt.

**25.** Verfahren zur Herstellung neuer Lysergsäureamide der allgemeinen Formel I nach Anspruch 1, oder einem der Ansprüche 13-22,dadurch gekennzeichnet, daß man Verbindungen der allgemeinen Formel

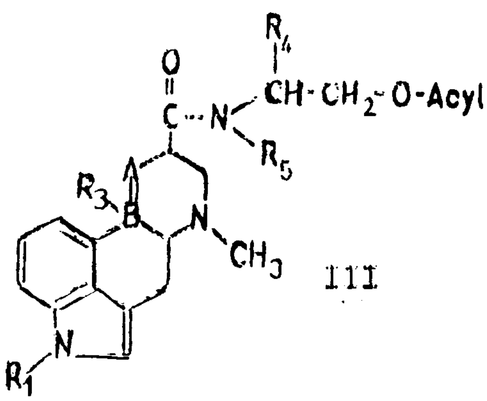

III

unter Verwendung eines milden Halogenierungsmittels nachträglich in eine Verbindung der Formel I, worin $R_2$ Halogen bedeutet, überführt.

**26.** Verfahren zur Herstellung neuer Lysergsäureamide der allgemeinen Formel I nach Anspruch 1, oder einem der Ansprüche 13-22,dadurch gekennzeichnet, daß man Zwischenprodukte der allgemeinen Formel V, worin $R_1$ Wasserstoff bedeutet und A...B für

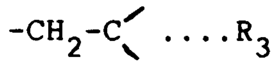

steht, mit Alkyl- bzw. Benzylhalogeniden in Verbindungen der allgemeinen Formel V umwandelt, worin $R_1$ Methyl oder Benzyl bedeuten und durch nachfolgende Acylierung die neuen Verbindungen der allgemeinen Formel I herstellt.

**27.** Verfahren zur Herstellung neuer Lysergsäureamide der allgemeinen Formel I nach Anspruch 1 oder einem der Ansprüche 13-22,dadurch gekennzeichnet, daß man Zwischenprodukte der allgemeinen Formel V, worin A...B für

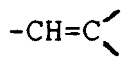

steht in saurem Milieu durch fotochemische Addition von Methanol in Derivate der allgemeinen Formel V, worin A...B für

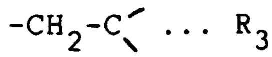

und $R_3$ für Methoxy steht, überführt und anschließend zu Verbindungen der allgemeinen Formel I acyliert.

**28.** Verfahren zur Herstellung neuer Lysergsäureamide der allgemeinen Formel I nach Anspruch 1, oder einem der Ansprüche 13-22,23,24,dadurch gekennzeichnet, daß zur Herstellung der aktivierten Formen der allgemeinen Formel II das Geiger-König-Verfahren oder das Dimethylformamid-Schwefeltrioxid-Verfahren Anwendung finden.

**29.** Verfahren zur Herstellung neuer Lysergsäureamide der allgemeinen Formel I nach Anspruch 1, oder einem der Ansprüche 13-22,23,26,27 dadurch gekennzeichnet, daß die Acylierung der gewonnenen Verbindungen der allgemeinen Formel IV und V zu I mit Hilfe eines Säurechlorids oder Säureanhydrids in Pyridin erfolgt.

**30.** Verfahren zur Herstellung neuer Lysergsäureamide der allgemeinen Formel I nach Anspruch 1, oder einem der Ansprüche 13-22,25 dadurch gekennzeichnet, daß als mildes Halogenierungsmittel N-Bromsuccinimid in Methylenchlorid oder Dioxan eingesetzt wird.

**31.** Verfahren zur Herstellung neuer Lysergsäureamide der allgemeinen Formel I nach Anspruch 1, oder einem der Ansprüche 13-22,26,dadurch dadurch gekennzeichnet, daß Alkylierung sowie Benzylierung vorteilhaft unter Eiskühlung in Dimethylformamid/Natriumhydroxid durchgeführt werden.

**32.** Pharmazeutische Zusammensetzungen enthaltend ein Lysergsäurederivat der allgemeinen Formel

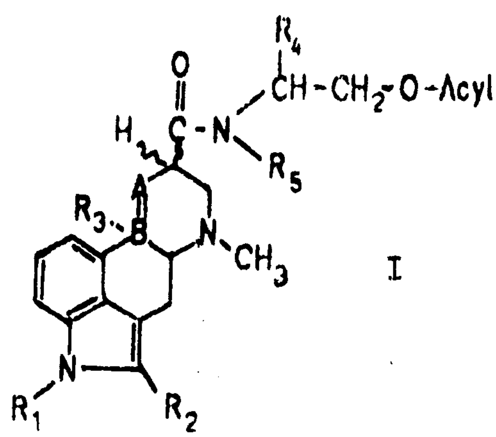

I

bzw. eines seiner physiologisch verträglichen Säureadditionssalze gemäß Anspruch 1, oder einem der Ansprüche 13-22 als Wirkstoff insbesondere zur Behandlung von

- postapoplektischen Zuständen
- M-inderung kognitiver und mnestischer Leistungen unterschiedlicher Genese
- altersbedingten, hirnorganischen Psychosyndromen
- vaskulär bedingten cerebralen Funktionsstörungen
- hypoxisch, neurotoxisch und traumatisch bedingten cerebralen Funktionsstörungen
- verminderter cerebraler Leistungsfähigkeit bei Alkoholikern
- pathologisch bzw. altersbedingt vermindertem Venentonus.

**33.** Nootropika in Form von Tabletten, Dragees, Tropflösungen, eines Ampullenpräparates oder anderer geeigneter Verabreichungsformen enthaltend 0,1 bis 10 mg pro Dosierungseinheit eines Lysergsäurederivates der allgemeinen Formel I bzw. eines seiner physiolog. verträglichen Säureadditionssalze nach Anspruch 1, oder einem der Ansprüche 13-22 als Wirkstoff neben üblichen galenischen Beimengungen, wie z. B. Trägerstoffen, Antioxidantien und Verdünnungsmitteln.

## Claims

**1.** Lysergic acid amides having the general formula

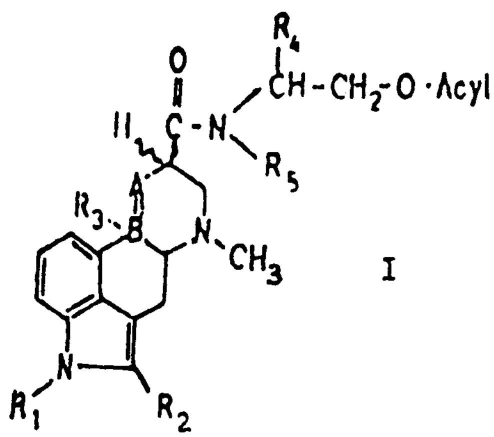

I

where A ... B stand for the bonds

$$-CH=C< \quad \text{or} \quad -CH_2-C< \ldots R_3$$

and the radicals are as follows:

$R_1$ denotes hydrogen, lower alkyl or benzyl, optionally monosubstituted by halogen,

$R_2$ denotes hydrogen or halogen,

$R_3$ denotes hydrogen or alkoxy,

$R_4$ denotes hydrogen or lower alkyl,

$R_5$ denotes hydrogen or $-CH_2-CH_2-O$-acyl, but $R_4$ and $R_5$ do not simultaneously denote hydrogen, and

acyl is an aliphatic acid radical,

the lysergic acid or isolysergic acid form being present, depending on the position of the two substituents at the $C_8$ of the ergoline skeleton relative to the hydrogen atom on the $C_5$, and salts thereof with physiologically compatible acids.

**2.** 9,10-dihydrolysergic acid-bis-(β-acetoxyethyl)-amide according to claim 1.

**3.** 2-bromo-9,10-dihydrolysergic acid-bis-(β-acetoxyethyl)-amide according to claim 1.

**4.** 1-methyl-9,10-dihydrolysergic acid-bis-(β-acetoxyethyl)-amide according to claim 1.

**5.** 10α-methoxy-9,10-dihydrolysergic acid-bis-(β-acetoxyethyl)-amide according to claim 1.

**6.** 1-methyl-9,10-dihydrolysergic acid-bis-(β-propionylethyl)-amide according to claim 1.

**7.** Lysergic acid-bis-(β-acetoxyethyl)-amide according to claim 1.

**8.** Isolysergic acid-bis-(β-acetoxyethyl)-amide according to claim 1.

**9.** 1-(3'-fluorobenzyl)-9,10-dihydrolysergic acid-bis-(β-acetoxyethyl)-amide according to claim 1.

**10.** 10 α-methoxy-9,10-dihydrolysergic acid-(+)-propanolamide-(2)-propionate according to claim 1.

**11.** 1-methyl-10α-methoxy-9,10-dihydrolysergic acid-(+)-propanolamide-(2)-acetate according to claim 1.

**12.** 1-methyl-lysergic acid-bis-(β-acetoxyethyl)-amide according to claim 1.

**13.** 9,10-dihydrolysergic acid-(+)-propanolamide-(2)-4'-chlorophenoxy acetate.

**14.** 9,10-dihydrolysergic acid-bis-(β-benzoylethyl)-amide.

**15.** 9,10-dihydrolysergic-(+)-propanolamide-(2)-3'-fluorobenzoate.

**16.** Lysergic acid-bis-(β-3'-fluorobenzoyl ethyl)-amide.

**17.** 1-benzyl-10α-methoxy-9,10-dihydrolysergic acid-bis-(β-3'-chlorobenzoyl ethyl)-amide.

**18.** 1-(3'-fluorobenzyl)-9,10-dihydrolysergic acid-bis-(β-3''-trifluoro-methyl benzoylethyl)-amide.

**19.** 10α-methoxy-9,10-dihydrolysergic acid-(+)-propanolamide-(2)-acetyl salicylate.

**20.** 10α-methoxy-9,10-dihydrolysergic acid-(+)-propanolamide-(2)-3'-chlorobenzoate.

**21.** 10α-methoxy-9,10-dihydrolysergic acid-(+)-butanolamide-(2)-2'-fluorobenzoate.

**22.** 10α-methoxy-9,10-dihydrolysergic acid-bis-(β-5'-bromonicotinyl ethyl)-amide.

**23.** A method of preparing novel lysergic acid amides having the general formula I according to claim 1 or any of claims 13 - 22, characterised in that a suitably substituted lysergic acid having the general formula

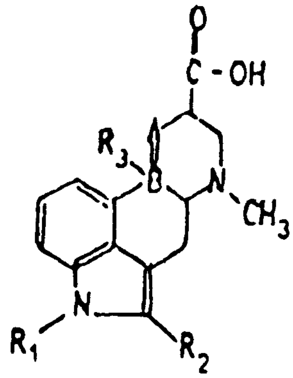

II

is first reacted by the conventional methods of ergoline chemistry, using an activated form, with an amine having the general formula

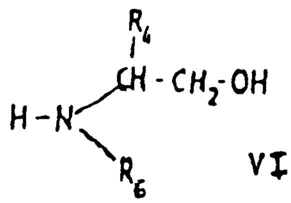

VI

to obtain intermediates having the general formula

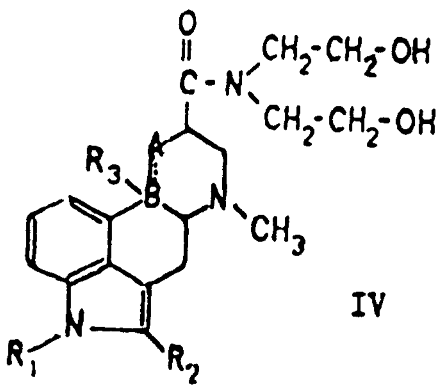

IV

OR

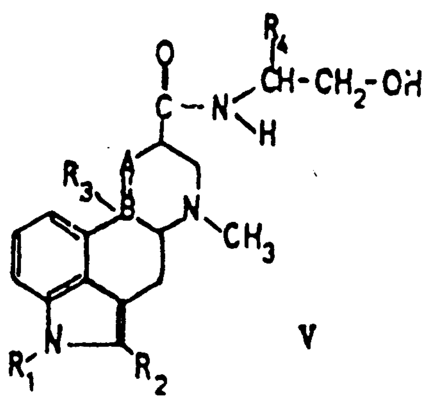

V

which are then converted by known methods to a compound having the general formula I.

**24.** A method of preparing novel lysergic acid amides having the general formula I according to claim 1 or any of claims 13 - 22, characterised in that a novel compound having the general formula I is obtained from a suitably substituted lysergic acid having the general formula II using an activated form and reacting it with an amine having the general formula

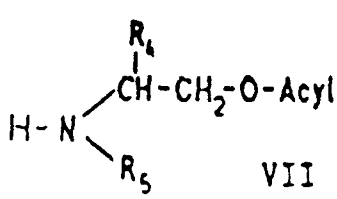

VII

**25.** A method of preparing novel lysergic acid amides having the general formula I according to claim 1 or any of claims 13 - 22, characterised in that compounds having the general formula

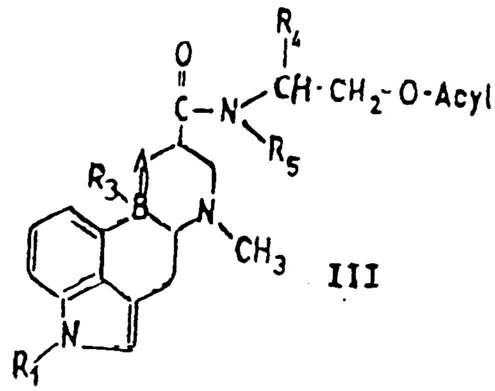

III

are subsequently converted, using a gentle halogenation agent, into a compound of formula I, in which $R_2$ denotes halogen.

**26.** A method of preparing novel lysergic acid amides having the general formula I according to claim 1 or any of claims 13 - 22, characterised in that intermediates having the general formula V, where $R_1$ denotes hydrogen and A ... B stands for

$$-CH_2-C< \;...\; R_3$$

are reacted with alkyl or benzyl halides and converted to compounds having the general formula V, in which $R_1$ denotes methyl or benzyl, and are then acylated to obtain the novel compounds having the general formula I.

**27.** A method of preparing novel lysergic acid amides having the general formula I according to claim 1 or any of claims 13 to 22, characterised in that intermediate products having the general formula V, where A ... B stands for

$$-CH=C< \;,$$

are converted in an acid medium, by photochemical addition of methanol, into derivatives having the general formula V, in which A ... B denotes

$$-CH_2-C< \;...\; R_3$$

and $R_3$ denotes methoxy, followed by acylation to compounds having the general formula I.

**28.** A method of preparing novel lysergic acid amides having the general formula I according to claim 1 or any of claims 13 - 22, 23, 24, characterised in that the Geiger-König method or the dimethyl formamide and sulphur trioxide method is used to obtain the activated forms having the general formula II.

**29.** A method of preparing novel lysergic acid amides having the general formula I according to claim 1 or any of claims 13 - 22, 23, 26, 27, characterised in that the resulting compounds having the general formulae IV and V are acylated to I, using an acid chloride or acide anhydride in pyridine.

**30.** A method of preparing novel lysergic acid amides having the general formula I according to claim 1 or any of claims 13 - 22, 25, characterised in that the gentle halogenation agent used is N-bromosuccinimide in methylene chloride or dioxane.

**31.** A method of preparing novel lysergic acid amides having the general formula I according to claim 1 or any of claims 13 - 22, 26, characterised in that alkylation and benzylation are preferably carried out with ice cooling in dimethyl formamide and sodium hydroxide.

**32.** Pharmaceutical compositions in which the active principle is a lysergic acid derivative having the general formula

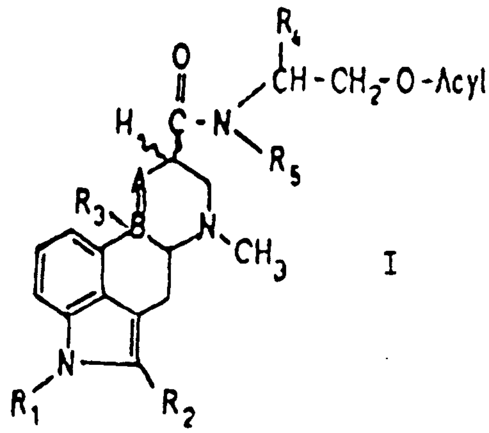

I

or one of its physiologically compatible acid addition salts according to claim 1 or any of claims 13 - 22, more particularly for treatment of

- post-apoplectic states
- impaired cognition and memory, due to various causes,
- organic brain psychosyndromes due to ageing,
- vascular cerebral functional disorders,
- hypoxic, neurotoxic and traumatic cerebral functional disorders,
- reduced brain capacity in alcoholics, or
- reduced vein tone, whether pathological or due to ageing.

**33.** Nootropics in the form of tablets, dragees, drip solutions, ampulla preparations or other suitable forms of administration, in which each unit dose contains 0.1 to 10 mg of the active principle, i.e. a lysergic acid derivative having the general formula I or one of its physiologically compatible acid addition salts according to claim 1 or any of claims 13 - 22, together with conventional galenic admixtures such as excipients, anti-oxidising agents and diluents.

**Revendications**

**1.** Lysergamides de formule générale

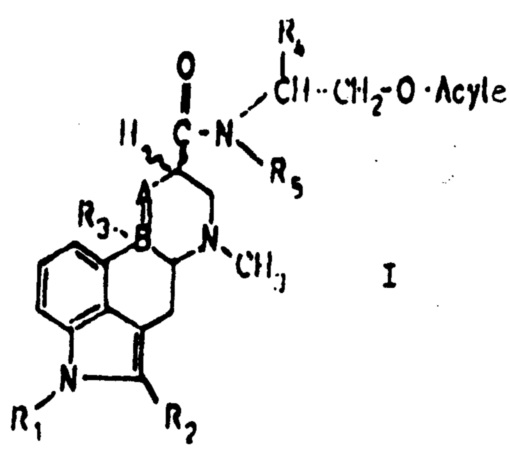

I

dans laquelle A ...B représente les liaisons

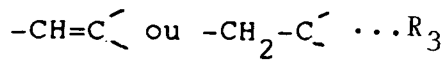

et les restes signifient

$R_1$ l'hydrogène, un alkyle inférieur ou un benzyle qui est éventuellement monosubstitué par un halogène,

$R_2$ l'hydrogène ou un halogène,

$R_3$ l'hydrogène ou un alcoxy,

$R_4$ l'hydrogène ou un alkyle inférieur,

$R_5$ l'hydrogène ou $-CH_2-CH_2-O-$acyle, $R_4$ et $R_5$ n'étant cependant pas en même temps de l'hydrogène,

acyle un reste acide, et dans laquelle, selon la position des deux substituants en $C_8$ du squelette ergoline par rapport à l'atome d'hydrogène en $C_5$, aussi bien la forme acide lysergique que la forme acide isolysergique peuvent être présentes, ainsi que leurs sels avec des acides physiologiquement compatibles.

**2.** Bis-($\beta$-acétoxyéthyl)-amide de l'acide 9,10-dihydrolysergique selon la revendication 1.

**3.** Bis-($\beta$-acétoxyéthyl)-amide de l'acide 2-bromo-9,10-dihydrolysergique selon la revendication 1.

**4.** Bis-($\beta$-acétoxyéthyl)-amide de l'acide 1-méthyl-9,10-dihydrolysergique selon la revendication 1.

**5.** Bis-($\beta$-acétoxyéthyl)-amide de l'acide 10$\alpha$-méthoxy-9,10-dihydrolysergique selon la revendication 1.

**6.** Bis-($\beta$-propionyléthyl)-amide de l'acide 1-méthyl-9,10-dihydrolysergique selon la revendication 1.

**7.** Bis-($\beta$-acétoxyéthyl)-amide de l'acide lysergique selon la revendication 1.

**8.** Bis-($\beta$-acétoxyéthyl)-amide de l'acide isolysergique selon la revendication 1.

**9.** Bis-($\beta$-acétoxyéthyl)-amide de l'acide 1-(3'-fluorobenzyl)-9,10-dihydrolysergique selon la revendication 1.

**10.** Propionate de (+)-propanolamide-(2) de l'acide 10$\alpha$-méthoxy-9,10-dihydrolysergique selon la revendication 1.

**11.** Acétate de (+)-propanolamide-(2) de l'acide 1-méthyl-10$\alpha$-méthoxy-9,10-dihydrolysergique selon la revendication 1.

**12.** Bis-($\beta$-acétoxyéthyl)-amide de l'acide 1-méthyllysergique selon la revendication 1.

**13.** 4'-chlorophénoxyacétate de (+)-propanolamide-(2) de l'acide 9,10-dihydrolysergique.

**14.** Bis($\beta$-benzoyléthyl)-amide de l'acide 9,10-dihydrolysergique.

**15.** 3'-fluorobenzoate de (+)-propanolamide-(2) de l'acide 9,10-dihydrolysergique.

**16.** Bis-($\beta$-3'-fluorobenzoyléthyl)-amide de l'acide lysergique

**17.** Bis-($\beta$-3'-chlorobenzoyléthyl)-amide de l'acide 1-benzyl-10$\alpha$-méthoxy-9,10-dihydrolysergique.

**18.** Bis-($\beta$-3''-trifluorométhylbenzoyléthyl)-amide de l'acide 1-(3'-fluorobenzyl)-9,10-dihydrolysergique.

**19.** Acétylsalicylate de (+)-propanolamide-(2) de l'acide 10$\alpha$-méthoxy-9,10-dihydrolysergique.

**20.** 3'-chlorobenzoate de (+)-propanolamide-(2) de l'acide 10$\alpha$-méthoxy-9,10-dihydrolysergique.

**21.** 2'-fluorobenzoate de (+)-butanolamide-(2) de l'acide 10α-méthoxy-9,10-dihydrolysergique.

**22.** Bis-(β-5'-bromonicotinyléthyl)-amide de l'acide 10α-méthoxy-9,10-dihydrolysergique.

**23.** Procédé pour la préparation de nouveaux lysergamides de formule générale I selon la revendication 1 ou l'une des revendications 13 à 22, caractérisée en ce qu'on fait réagir un acide lysergique convenablement substitué, répondant à la formule générale

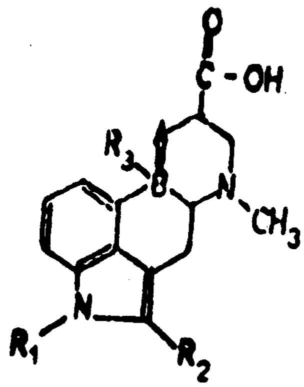

II

à l'aide des méthodes habituelles de la chimie de l'ergoline, en passant par une forme activée, avec une amine de formule générale

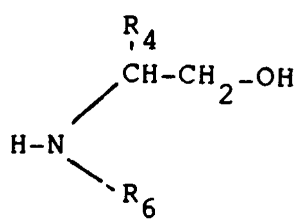

VI

tout d'abord en les produits intermédiaires de formule générale

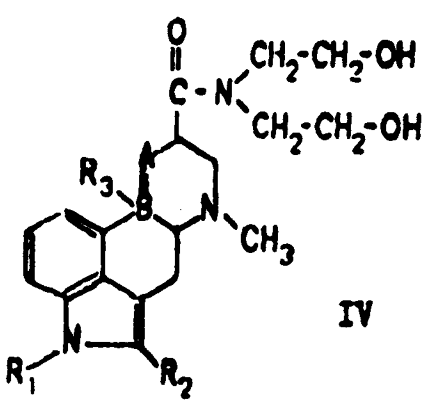

IV

respect.

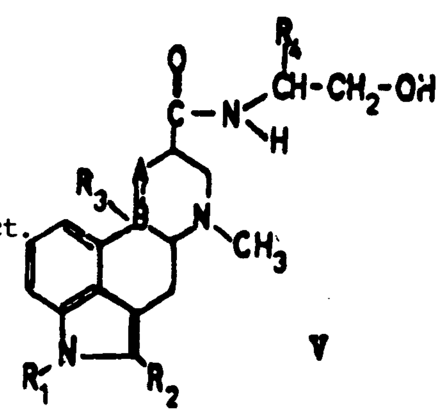

V

et que l'on transforme ensuite ceux-ci, selon des procédés connus, en un composé de formule générale I.

**24.** Procédé pour la préparation de nouveaux lysergamides de formule générale I selon la revendication 1 ou l'une des revendications 13 à 22, caractérisé en ce que l'on obtient à partir d'un acide lysergique convenablement substitué de formule générale II, avec une amine de formule générale

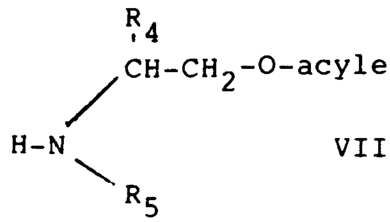

VII

en passant par une forme activée, un nouveau composé de formule I.

**25.** Procédé pour la préparation de nouveaux lysergamides de formule générale I selon la revendication 1 ou l'une des revendications 13 à 22, caractérisé en ce que l'on transforme ultérieurement des composés de formule générale

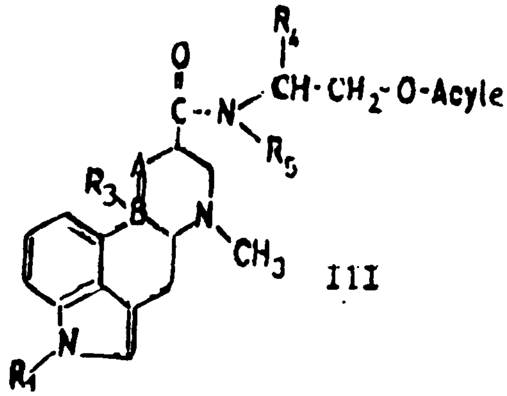

III

en utilisant un agent d'halogénation doux, en un composé de formule I où $R_2$ signifie un halogène.

**26.** Procédé pour la préparation de nouveaux lysergamides de formule générale I selon la revendication 1 ou l'une des revendications 13 à 22, caractérisé en ce que l'on transforme des produits intermédiaires de formule générale V dans laquelle $R_1$ signifie l'hydrogène et A...B représente

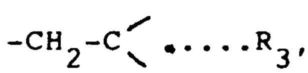

avec des halogénures d'alkyle ou de benzyle, en des composés de formule générale V où $R_1$ signifie un méthyle ou un benzyle et que l'on prépare par une acylation subséquente les nouveaux composés de formule générale I.

**27.** Procédé pour la préparation de nouveaux lysergamides de formule générale I selon la revendication 1 ou l'une des revendications 13 à 22, caractérisé en ce que l'on transforme des produits intermédiaires de formule générale V dans laquelle A...B représente

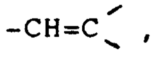

en milieu acide par addition photochimique de méthanol, en des dérivés de formule générale V où A...B représente

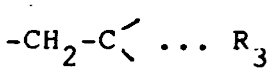

et $R_3$ représente un méthoxy et qu'on les acyle subséquemment en des composés de formule

générale I.

**28.** Procédé pour la préparation de nouveaux lysergamides de formule générale I selon la revendication 1 ou l'une des revendications 13 à 22, 23, 24, caractérisé en ce que, pour la préparation des formes activées de formule générale II, il est fait appel au procédé Geiger-König ou au procédé au diméthylformamide-anhydride sulfurique.

**29.** Procédé pour la préparation de nouveaux lysergamides de formule générale I selon la revendication 1 ou l'une des revendications 13 à 22, 23, 26,27, caractérisé en ce que l'acylation en I des composés de formules générales IV et V obtenus s'effectue à l'aide d'un chlorure d'acide ou d'un anhydride d'acide dans la pyridine.

**30.** Procédé pour la préparation de nouveaux lysergamides de formule générale I selon la revendication 1 ou l'une des revendications 13 à 22, 25, caractérisé en ce qu'on utilise comme agent d'halogénation doux du N-bromosuccinimide dans du chlorure de méthylène ou du dioxanne.

**31.** Procédé pour la préparation de nouveaux lysergamides de formule générale I selon la revendication 1 ou l'une des revendications 13 à 22, 26, caractérisé en ce que l'alkylation ainsi que la benzylation s'effectuent avantageusement, sous refroidissement par de la glace, dans le mélange diméthylformamide/hydroxyde de sodium.

**32.** Compositions pharmaceutiques contenant un dérivé d'acide lysergique de formule générale

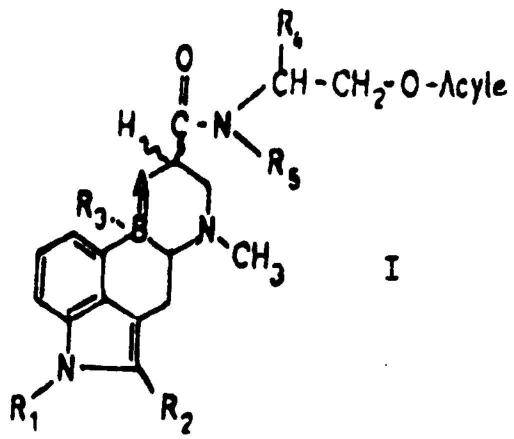

I

ou un de ses sels d'addition d'acides physiologiquement compatibles selon la revendication 1 ou l'une des revendications 13 à 22 en tant que principe actif, notamment pour le traitement

- des états postapoplectiques
- de la diminition des facultés cognitives et mnésiques d'origines diverses
- des syndromes psychotiques liés à la sénéscence
- des troubles fonctionnels cérébraux d'origine vasculaire
- des troubles fonctionnels cérébraux d'origine anoxique, nemotoxique et traumatique
- de la diminution de la capacité cérébrale chez les alcooliques
- de la diminution du tonus veineux pathologique ou liée à la sénéscence.

**33.** Nootropes sous forme de comprimés, de dragées, de gouttes, d'une préparation en ampoules ou d'autres formes d'administration appropriées contenant, à titre de principe actif, 0,1 à 10 mg par dose unitaire d'un dérivé de l'acide lysergique de formule générale I ou d'un de ses sels d'addition d'acide physiologiquement compatibles selon la revendication 1 ou l'une des revendications 13 à 22, à côté d'adjuvants galéniques usuels, tels que par ex. des véhicules, des antioxydants et des diluants.